# EUROPEAN PATENT APPLICATION

(11) **EP 2 338 461 A1**
(43) Date of publication of application: **29.06.2011**
(21) Application number: 09306306.3
(22) Date of filing: 22.12.2009
(51) Int. Cl.: A61K 8/04, A61K 8/81, A61Q 1/10, A61Q 1/12

(54) **Colored micronized spherical polymer powder and cosmetic composition thereof**

(71) Applicant: DUPONT POLYMER POWDERS SWITZERLAND SARL, 1630 Bulle (CH)
(72) Inventor: Magnin, Olivier, CH-1005 Lausanne (CH); Gherardi, Carole, 01710 Thoiry (FR); Raspail, Vincent, 01210 Ferney-Voltaire (FR); Granjou, Ludovic, 1800 Vevey (CH)
(74) Representative: Biering, Christine

(57) **Abstract**

The present invention provides a colored micronized polymer powder for the use in cosmetics, the powder comprising at least one thermoplastic polymer wherein the particles of the colored micronized polymer powder having a spherical shape and an average particle size in the range of 0.1 to 100 µm. The present invention provides also a cosmetic composition comprising the at least one colored micronized polymer powder according to the invention.

The powder according to the invention makes it possible to provide ultra-fine colored micronized polymer powders having a desired coloration as well as high-performed voluminizing effect, soft focus effect, mattifying effect and long lasting effect in cosmetic end-use applications such as mascaras and make-up.

## Description

### Field of the Invention

The invention is directed to a colored micronized spherical polymer powder comprising at least one thermoplastic polymer for the application in cosmetics as well as to a process of preparation the colored micronized spherical polymer powder and to a cosmetic composition thereof.

### Description of Prior Art

Micronized polymer powders can be widely used as additives and components in the cosmetic industry, due to their main properties regarding optical effects, skin feeling effects, absorption of skin sebum and mechanical effects. Particularly microspheres based on micronized polymer powders may provide such properties. Optical effects may comprise effects on soft focus, optical blurring and matt look which can provide a more natural appearance. Skin feeling effects may be provided based on modified tactile properties and reduced tackiness of the products. Furthermore, the compaction of, for example make-up powders, can be eased and the use of liquid binders can be decreased or avoided.

The known cosmetic preparations based on micronized polymer powders commonly include specific pigments such as black pigments which are difficult to handle and which, therefore, may cause problems on the above mentioned cosmetic effects.

Also, in general, micronized polymer powders are made from polymers based on crude oil. As known, the use of petrochemicals is critical because of decreased oil resources, increased processing costs, increased energy consumption and production of carbon dioxide harmful to the environment. Also, public concern about the health hazards arising from healthcare and cosmetic products which are prepared from crude oil.

Therefore, there is a need for improved micronized polymer powders for the application in cosmetics offering ultra-fine micronized polymer powders providing a wide range of desired colors by maintaining and/or improving cosmetic properties.

### Summary of the Invention

The present invention provides a colored micronized polymer powder for the use in cosmetics, the powder comprising at least one thermoplastic polymer wherein the particles of the colored micronized polymer powder having a spherical shape and an average particle size in the range of 0.1 to 100 µm.

The present invention provides also a cosmetic composition comprising the at least one colored micronized polymer powder composition according to the invention.

The colored micronized spherical polymer powder according to the invention makes it possible to provide ultra-fine colored micronized polymer powders having a desired coloration as well as high-performed voluminizing effect, soft focus effect, mattifying effect and long lasting effect in cosmetic end-use applications such as mascaras and make-up. Furthermore, the colored micronized polymer powder according to the invention allows to ease the powder compaction for make-up with high homogeneous result.

### Detailed Description of the Invention

The features and advantages of the present invention will be more readily understood, by those of ordinary skill in the art, from reading the following detailed description. It is to be appreciated those certain features of the invention, which are, for clarity, described above and below in the context of separate embodiments, may also be provided in combination in a single embodiment. Conversely, various features of the invention that are, for brevity, described in the context of a single embodiment, may also be provided separately or in any sub-combination. In addition, references in the singular may also include the plural (for example, "a" and "an" may refer to one, or one or more) unless the context specifically states otherwise.

All patents, patent applications and publications referred to herein are incorporated by reference in their entirety.

The colored micronized polymer powder according to the invention for the use in cosmetics is a powder comprising at least one thermoplastic polymer.

The term "thermoplastic" stated in the present description is related to the fact that the polymer can be repeatedly, in opposition to thermosets, melted and solidified by heating and cooling without involving any important changes in properties.

The thermoplastic polymer can be at least one non-functionalized and/or functionalized polymer, the functionalized polymer meaning that it is grafted and/or copolymerized with organic functionalities. It may be functionalized with acid, anhydride and/or epoxide functionalities.

Examples of the at least one thermoplastic polymer are thermoplastic functionalized polyesters, thermoplastic functionalized polyamides, thermoplastic functionalized poly(meth)acrylates, thermoplastic functionalized polyurethanes, thermoplastic functionalized polysiloxanes and thermoplastic functionalized and non-functionalized polyolefines. These thermoplastic polymers are known, as such, at a skilled person. Preferred is the use of thermoplastic functionalized polyolefines.

Examples of the acids and anhydrides used to functionalize the polymer, which may be mono-, di- or polycarboxylic acids are acrylic acid, methacrylic acid, maleic acid, fumaric acid, furnaric acid, itaconic acid, crotonic acid, itaconic anhydride, maleic anhydride and substituted maleic anhydride, e.g. dimethyl maleic anhydride or citrotonic anhydride, nadic anhydride, nadic methyl anhydride, and tetrahydrophthalic anhydride, or combinations of two or more thereof, maleic anhydride being preferred. Examples of epoxides used to functionalize the polymer are unsaturated epoxides comprising from four to eleven carbon atoms, such as glycidyl (meth)acrylate, allyl glycidyl ether, vinyl glycidyl ether and glycidyl itaconate, glycidyl (meth)acrylates being particularly preferred.

When one or more acid-functionalized polymers are used, they preferably contain from 0.05 to 25 wt-% of an acid, the wt-% being based on the total weight of the functionalized polymer.

When one or more anhydride-functionalized polymers are used, they preferably contain from 0.05 to 10 wt-% of an anhydride, the wt-% being based on the total weight of the functionalized polymers.

When one or more epoxide-functionalized polymers are used, they preferably contain from 0.05 to 15 wt-% of an epoxide, the wt-% being based on the total weight of the functionalized polymers.

The term "(meth) acrylic" stated in the present description is respectively intended to mean acrylic and/or methacrylic.

The particles of the colored micronized polymer powder of the invention may have an average particle size in the range of 0.1 to 100 µm. The average particle size can be selected in a range desired for the specific application in the cosmetics. Preferred is an average particle size in a range of 0.5 to 20 µm.

For example, the particles of the colored micronized polymer powder have a particle size distribution of D90 value less than or equal to 200 µm, more preferably between 20 and 75 µm.

The "D90 value" stated in the present description corresponds to a particle size below which 90 wt-% of the particles lie, wherein the particle size analysis is done by a laser diffraction method and meets the standards set forth in ISO 13320-1. The measurement can be done on a Malvern Mastersizer 2000.

The particles of the colored micronized polymer powder of the invention have a spherical shape.

The term "spherical shape" stated in the present description means that more than 80%, preferably more than 90%, of the particles have a spherical shape which means a globular shape, the term as known in the art.

The term "spherical polymer powder" stated in the present description means a powder of particles having a spherical shape as mentioned above.

Polyesters useful as thermoplastic functionalized polymers of this invention are based on at least one diol and at least one dicarboxylic acid selected from the group consisting of cyclic and branched aliphatic dicarboxylic acids having 4-12 carbon atoms and aromatic dicarboxylic acids having 8-12 carbon atoms. Examples of such polyesters are polyalkylene terephthalate, for example trimethylene terephtalate, or polyesters produced by polymerization between at least one diol and at least one carboxylic acid such butanedioic acid, pentanedioic acid, hexanedioic acid, dodecanedioic acid, and 1,4-cyclo-hexanedicarboxylic acid, terephthalic acid, isophthalic acid and 2,6-naphthalenedicarboxylic acid. The at least one diol can be a linear, cyclic, and/or branched aliphatic diol having 2-8 carbon atoms, preferably 2-5 carbon atoms. Examples of such diols are ethanediol, propanediol, 1,4-butanediol, 3-methyl-1,5-pentanediol, 2,2-dimethyl-1,3-propanediol, 2-methyl-1,3-propanediol, and 1 ,4-cyclohexanediol. The diol can also be at least one bio-based diol.

The term "bio-based diol" stated in the present description means a diol that is derived from agricultural corn by processes known in the art. In principle, such processes comprise the steps of harvesting the corn, getting sugar from the corn and transferring the sugar into the alcohol (diol) by fermentation. An example of a polyester based on bio-based diol is Sorona® 3 GTX of the company DuPont.

The thermoplastic polyester according to the invention can be prepared by processes known in the art.

The thermoplastic polyolefines useful as thermoplastic functionalized and/or non-functionalized polymers of this invention can be, for example, thermoplastic polyethylenes, polypropylenes, ethylene acid copolymers, ionomeric polymers, ethylene vinyl acetate (EVA) copolymers and ethylene alkyl (meth)acrylate copolymers.

Polyethylenes are known in the art and they are commonly available polyethylene resins selected from ultra low density polyethylene (ULDPE), very low density polyethylene (VLDPE), low density polyethylene (LDPE), linear low density polyethylene (LLPE), high density polyethylene (HDPE), known to those skilled in the art, metallocene polyethylene (mPE) and/or copolymers such as for example ethylene propylene copolymers and copolymers based on ethylene, propylene and EPDM. EPDM is a terpolymer of ethylene, at least one alpha-olefin, and a copolymerizable non-conjugated diene such as norbornadiene, 5-ethylidene-2-norbornene, dicyclopentadiene, 1,4-hexadiene and the like.

Polypropylenes may include homopolymers, random copolymers, block copolymers and terpolymers of propylene, known to those skilled in the art. Copolymers of propylene include copolymers of propylene with other olefins such as 1-butene, 2-butene and the various pentene isomers. Ethylene alpha-olefins copolymers comprise ethylene and one or more alpha-olefins. Examples of alpha-olefins include but are not limited to propylene, 1-butene, 1-pentene, 1-hexene-1, 4-methyl 1-pentene, 1-heptene, 1-octene, 1-nonene, 1-decene, 1-undecene and 1-dodecene.

Ethylene acid copolymers are made via free radical polymerization of ethylene and of one or more α,β-ethylenically unsaturated carboxylic acids and/or their anhydrides which may comprise 3 to 8 carbon atoms such as acrylic acid (AA), methacrylic acid (MAA), maleic acid monoethylester (MAME), also, for example, itaconic, maleic, fumaric acids and/or their anhydrides. The ethylene acid copolymers may optionally contain a third monomer, which can be a softening monomer. The term softening monomer is known to those skilled in the art and is used for softening polymers/copolymers. This softening monomer can decrease the crystallinity of the ethylene acid copolymer. Suitable softening monomers can be selected, for example, from alkyl acrylates and alkyl methacrylates, wherein the alkyl groups have from 1 to 8 carbon atoms.

The ethylene acid copolymers can thus be described as E/X/Y copolymers, wherein E represents copolymerized units of ethylene, X represents copolymerized units of the α,β-ethylenically unsaturated carboxylic acid, and Y represents copolymerized units of the softening monomer. The amount of X in the ethylene acid copolymer is from 1 to 30 wt-%, preferably from 9 to 25 wt-%, more preferably from 12 to 22 wt-%, and the amount of Y is from 0 to 30 wt-%, preferably 2 to 15 wt-%, more preferably from 4 to 12 wt-%, based on the total weight of the ethylene acid copolymer. The remainder of the copolymer comprises or consists essentially of copolymerized units of ethylene.

Alternatively, softening of ethylene acid copolymers can be done by adding one or more ethylene alkyl (meth)acrylate copolymers to the ethylene acid copolymers.

Preferred are ethylene acid copolymers in which Y is 0% of the copolymer. Therefore, E/X dipolymers comprising copolymerized residues of ethylene and of one or more α,β-ethylenically unsaturated carboxylic acids comprising from 3 to 8 carbon atoms are preferred. Specific examples of these preferred ethylene acid copolymers include, without limitation, ethylene acrylic acid copolymer (EAA), ethylene methacrylic acid copolymer (EMAA) ethylene maleic acid monoethylester copolymer (EMAME) or mixtures thereof.

Methods of preparing ethylene acid copolymers are known to those skilled in the art. Ethylene acid copolymers can be prepared in continuous polymerizers by use of "co-solvent technology" as described in US 5,028,674 or by employing somewhat higher pressures than those at which copolymers with lower acid can be prepared.

Ionomeric polymers suitable according to this invention can be ethylene acid copolymers described above that contain metal ions in addition to the organic backbone. Such ionomeric polymers can be, for example, ionic copolymers of an olefin such as ethylene with partially neutralized α,β-unsaturated C₃-C₈ carboxylic acid. Preferably, the acid copolymer is acrylic acid (AA) or methacrylic acid (MAA).

Compounds suitable for neutralizing an ethylene acid copolymer include ionic compounds having basic anions and alkali metal cations (for example, lithium or sodium or potassium ions), transition metal cations (for example, zinc ion) or alkaline earth metal cations (for example magnesium or calcium ions) and mixtures or combinations of such cations. Ionic compounds that may be used for neutralizing the ethylene acid copolymers include alkali metal formates, acetates, nitrates, carbonates, hydrogen carbonates, oxides, hydroxides or alkoxides. Other useful ionic compounds include alkaline earth metal formates, acetates, nitrates, oxides, hydroxides or alkoxides of alkaline earth metals. Transition metal formates, acetates, nitrates, carbonates, hydrogen carbonates, oxides, hydroxides or alkoxides may also be used. Preferably, neutralizing agents are chosen among sources of sodium ions, potassium ions, zinc ions, magnesium ions, lithium ions, transition metal ions, alkaline earth metal cations and combinations of two or more thereof; more preferably zinc ions and combination of zinc ions, and still more preferably a combination of zinc ions and sodium ions or zinc ions and lithium ions.

The acid groups of the ionomeric polymers can be neutralized in a range of 10 to 90%, preferably 25 to 50 wt-% and more preferably 20 to 40 wt-%. The total amount of acid monomer and neutralized acid monomer in the ionomeric polymers is preferably between 12 and 25 wt-%, more preferably between 14 and 22 wt-%, and more preferably between 15 and 20 wt-%, the weight percentage being based on the total weight of the ionomeric polymer.

In analogy with the ethylene acid copolymers, ionomeric polymers can be described as E/X/Y copolymers where E is an olefin such as ethylene, X is a α,β-unsaturated C₃-C₈ carboxylic acid, and Y is a softening monomer, wherein X is from 12 to 25 wt-%, preferably from 14 to 22 wt-% of the E/X/Y copolymer and Y can be present in an amount of from about 0 to 30 wt-% of the E/X/Y copolymer, wherein the carboxylic acid functionalities are at least partially neutralized.

Softening of the ionomeric polymers can be done by adding one or more ethylene alkyl (meth)acrylate copolymers to the ionomeric polymer.

Ionomeric polymers and their methods of manufacture are described for example in US 3,264,272.

Furthermore, ionomeric polymers suitable according to this invention can be ionic copolymers of an olefin other than ethylene with partially neutralized α,β-unsaturated C₃-C₈ carboxylic acid. Such ionomeric polymers and their methods of manufacture are described, for example, in US 3933954.

EVA copolymers preferably comprise an amount of copolymerized vinyl acetate units from 5 to 40 wt-%, preferably from 10 to 30 wt-% and more preferably from 15 to 25 wt-%, the wt-% being based on the total weight of the functionalized polyolefin. A mixture of two or more different EVA copolymers can be used as components of the thermoplastic functionalized polyolefin.

Ethylene alkyl (meth)acrylate copolymers are thermoplastic ethylene copolymers derived from the copolymerization of ethylene comonomer and at least one alkyl (meth)acrylate comonomer. "Alkyl (meth)acrylate" refers to alkyl acrylate and/or alkyl methacrylate. The alkyl group of the ethylene alkyl (meth)acrylate copolymer contains from one to ten carbon atoms and preferably from one to four carbon atoms, i.e. preferred copolymers are ethylene methyl (meth)acrylate copolymers, ethylene ethyl (meth)acrylate copolymers, ethylene butyl (meth)acrylate copolymers. Functionalized ethylene alkyl (meth)acrylate preferably comprises a relative amount of copolymerized alkyl (meth)acrylate units from 0.1 to 45 wt-%, preferably from 5 to 35 wt-% and still more preferably from 8 to 28 wt-%, the weight percentage being based on the total weight of the functionalized polyolefin.

The use of ionomeric polymers as the at least one thermoplastic functionalized polyolefin in the powder of the invention is preferred.

The colored micronized spherical polymer powder according to the invention may be prepared by forming a mixture of the melted or soften thermoplastic functionalized polymer in an aqueous medium, optionally with at least one surfactant and with further components such as pigments and/or other coloring agents, fillers, extenders, modifiers and/or other additives. The mixture is sheared in a shear device under a pressure of, for example, 1 to 100 bars, at a temperature above the melting point of the particular thermoplastic functionalized polymer used, under forming a homogeneous mixture, until the polymer particle size is reduced to within the desired average particle size range, less than or equal to 100 µm, as mentioned above, providing a spherical shape to the particles.

The temperature can be in the range of, for example, 50 to 300°C.

The resulted slurry of particles is then cooled from a temperature above the melting point of the polymer to a temperature below the polymer freezing point. Sufficient pressure is maintained throughout the system to prevent boiling of the aqueous medium; the pressure can be, for example, in the range of 1 to 100 bars. The water, surfactants and the polymer are constantly agitated in regions of the process where turbulent flow conditions do not exist thereby preventing separation of the polymer and aqueous media into two layers. The polymer particles are separated from the aqueous medium by conventional techniques such as filtration or centrifugation. The wet powder is then dried by conventional methods known in the art, for example, flash drying, rotary drying.

The colored micronized polymer powder according to the invention may comprise the at least one thermoplastic functionalized polymer in amounts in the range of 40 to 99 wt-%, preferably 60 to 99 wt-%, based on the total weight of the colored micronized powder of the invention.

Examples of the surfactants are, for example, ionic, non-ionic surfactants and a combination thereof, known in the art. Suitable ionic surfactants include, for example, ammonium lauryl sulphate, sodium lauryl sulphate and sodium dodecyl benzene sulfonate as well as ionic surfactant formed in situ by the reaction of one or more of ammonium hydroxide, triethanolamine, morpholine and dimethyl ethanolamine with the carboxyl functionality integral to the copolymer. Suitable non-ionic surfactants are polyoxypropylene-polyoxyethylene block copolymer, alkylphenol thioxylates, and/or ethyleneoxide-propylene glycol polymer. The amount of the surfactant in the above mentioned mixture can be in the range of 0.001 to 25 wt-%, preferably 0.01 to 10 wt-%, more preferably 0.01 to 5 wt-%, the wt-% based on the total weight of the thermoplastic functionalized polymer.

The colored micronized polymer powder according to the invention may also contain further components such as pigments and/or other coloring agents, fillers, extenders, modifiers and/or other additives known to those skilled in the art, for example in a range of 0.1 to 60 wt-%, preferred 5 to 40 wt%, based on the total weight of the colored micronized powder of the invention.

Examples of pigments and/ or other coloring agents are transparent pigments, color-imparting and/or special effect-imparting pigments and/or fillers. Suitable color-imparting pigments are pigments of an organic or inorganic nature, for example, titanium dioxide, iron oxide pigments, carbon black, phthalocyanine pigments, quinacridone pigments and pyrrolopyrrole pigments. Examples of special effect pigments are metal pigments, for example, of aluminum, copper or other metals, interference pigments, such as, for example, metal oxide-coated metal pigments, for example, iron oxide-coated aluminum, coated mica, such as, for example, titanium dioxide-coated mica, graphite effect-imparting pigments, iron oxide in flake form, liquid crystal pigments, coated aluminum oxide pigments, coated silicon dioxide pigments. Examples of fillers and/or extenders include, without limitation, silicon dioxide, silicate, such as, aluminum silicate, barium sulfate, calcium carbonate, magnesium carbonate and double carbonates of them and talc.

Modifiers and other additives include without limitation, plasticizers, impact modifiers, stabilizers including viscosity stabilizers and hydrolytic stabilizers, lubricants, antioxidants, UV light stabilizers, antifog agents, antistatic agents, flame retardant agents, and processing aids known in powder coating art like such as example antiblock agents, release agents, flow-control agents, flatting agents, and catalysts.

Further components can be further polymers different from the at least one thermoplastic functionalized polymers of the invention, for example, polyolefines, in a range of 0 to 30 wt%, based on the total weight of the colored micronized powder of the invention.

The colored micronized polymer powder may therefore comprise
A) 40 to 99 wt-% of the at least one thermoplastic functionalized polymer,
B) 0.1 to 60 wt-% of at least one pigment and/or other coloring agent, filler, extender, modifier and/or other additive, and
C) 0 to 30 wt-% of at least one polymer different from the at least one polymer of A),
the wt-% based on the total weight of the colored micronized powder, wherein the particles of the colored micronized polymer powder having a spherical shape and an average particle size in the range of 0.1 to 100 µm.

The colored micronized spherical polymer powder of the invention can be used as a component in cosmetic compositions in amounts in a range of, for example, 1 to 50 wt-%, preferably 2 to 10 wt-% of at least one colored micronized spherical polymer powder composition of the invention, the wt-% based on the total weight of the cosmetic composition. The amounts can be adapted with the specific cosmetic end-use application, for example, 2 to 5 wt-% for soft focus and mattifying effect, for example in make-up, and 5 to 10 wt-% for voluminizing effect and long lasting effect, for example, in mascaras.

Therefore, this invention also refers to a cosmetic composition comprising the at least one colored micronized polymer powder composition described above.

## Claims

1. A colored micronized polymer powder for the use in cosmetics, the powder comprising at least one thermoplastic polymer wherein the particles of the colored micronized polymer powder having a spherical shape and an average particle size in the range of 0.1 to 100 µm.

2. The powder according to claim 1 comprising
A) 40 to 99 wt-% of the at least one thermoplastic functionalized polymer,
B) 0.1 to 60 wt-% of at least one pigment and/or other coloring agent, filler, extender, modifier and/or other additive, and
C) 0 to 30 wt-% of at least one polymer different from the at least one polymer of A),
the wt-% based on the total weight of the colored micronized powder.

3. The powder according to claim 1 and 2 wherein the thermoplastic polymer is a thermoplastic functionalized polyolefine.

4. The powder according to claims 1 to 3 wherein the particles having an average particle size in the range of 0.5 to 20 µm.

5. The powder according to claims 1 to 4 wherein the particles having a particle size distribution of D90 value between 20 and 75 µm.

6. A method for preparation of the colored micronized polymer powder according to claims 1 to 5 by forming a mixture of the melted or soften thermoplastic polymer with the further components of the powder in an aqueous medium, sharing under pressure at a temperature above the melting point of the thermoplastic polymer until the polymer particle size is reduced within the desired average particle size range, and cooling the resulted slurry of particles.

7. A cosmetic composition comprising the at least one colored micronized polymer powder of claim 1 in a range of 1 to 50 wt-%, the wt-% based on the total weight of the cosmetic composition.
